# EUROPÄISCHE PATENTSCHRIFT

(11) **EP 0 521 089 B1**
(45) Veröffentlichungstag und Bekanntmachung des Hinweises auf die Patenterteilung: **15.02.1995**
(21) Anmeldenummer: 91907105.0
(22) Anmeldetag: 03.04.1991
(51) Int. Cl.: A61B 3/10, A61B 3/107, A61B 3/00

(54) **VORRICHTUNG ZUR ANALYSE DES TRÄNENFILMS**
DEVICE FOR ANALYSING THE LACHRYMAL FILM
DISPOSITIF POUR L'ANALYSE DU FILM LACRYMAL

(30) Priorität: 23.01.1991 DE 4101794
(43) Veröffentlichungstag der Anmeldung: 07.01.1993
(73) Patentinhaber: MUSTER, Inge, D-91325 Adelsdorf (DE); MUSTER, Wilfried H., D-91325 Adelsdorf (DE)
(72) Erfinder: MUSTER, Inge, D-91325 Adelsdorf (DE); MUSTER, Wilfried H., D-91325 Adelsdorf (DE)
(74) Vertreter: Göbel, Matthias, Dipl.-Ing.
(86) Internationale Anmeldenummer: EP9100632
(87) Internationale Veröffentlichungsnummer: WO9212666

(56) Entgegenhaltungen:
- EP-A- 0 109 846
- EP-A- 0 232 986
- EP-A- 0 397 962
- DE-A- 3 108 878
- DE-B- 1 211 814
- GB-A- 2 123 977
- US-A- 4 597 648

## Beschreibung

Die Erfindung betrifft eine Beleuchtungsvorrichtung zur Untersuchung des Tränenfilms eines Auges nach dem Oberbegriff von Anspruch 1.

Für die Beurteilung des Tränenfilms sind die Verwendung von Vier- bzw. Sechspunktringleuchten bekannt, z.B. aus US-A-4,597,648, deren Lichtpunkte im Abstand voneinander entlang einer Kreislinie auf einem ringförmigen Träger ausgebildet und auf das Auge projizierbar sind. Diesen Ringleuchten ist der Nachteil gemeinsam, daß sie eine Beurteilung des Tränenfilms nur in Bereichen ermöglichen, in denen der Tränenfilm relativ gleichmäßig ist.

Es ist Aufgabe der Erfindung eine Vorrichtung zu schaffen, die zur Montage an handelsüblichen Spaltlampen bestimmt ist und eine Beurteilung des Tränenfilms in Bereichen beliebiger Dicke ermöglicht.

Der Erfindung gemäß ist diese Aufgabe gelöst durch die Merkmale des kennzeichnenden Teils von Anspruch 1. Bevorzugt weist dabei der Ringkörper auf der Kreislinie sechs umd im Mittelpunktsbereich der Kreislinie eine Lichtaustrittsstelle auf. Der Tränenfilm ist vermittels der Vorrichtung sowohl in relativ gleichmäßigen Tränenfilmbereichen als auch den dünnsten Tränenfilmbereichen beurteilbar, wobei gemäß dem Erscheinungsbild der Interferenzmuster der Tränenfilm in verschiedene Gruppen aufteilbar ist. Hierdurch wird die Voraussetzung zu einer einfachen und sicheren Abstimmung des jeweiligen Tränenfilms mit dem für diesen am besten geeigneten Kontaktlinsenmaterial geschaffen, wodurch die Anpaßzeit von Kontaktlinsen stark verkürzt und ein längerer Anpaßerfolg erreichbar ist. Außerdem eignet sich die Vorrichtung zur Erkennung von Keratokonus, Fluorobildbeobachtung und Hornhautinspektion. Fernerhin ist die Vorrichtung für Vidio- und Fotodokumentation geeignet.

In Ausgestaltung der Vorrichtung ist der Trägerkörper durch einen Ringkörper gebildet, der bevorzugt durch ein Halteglied an einem eine gemeinsame Lichtquelle für die Lichtaustrittsstellen aufweisenden Gehäuse festgelegt ist und bei dem die Strahlung der Lichtquelle über optische Lichtleiter den Lichtaustrittsstellen im Ringkörper zuführbar ist.

Es versteht sich, daß die Längsmittelachsen der Lichtaustrittsstellen parallel verlaufen können. Zweckmäßig sind jedoch die auf der Kreislinie ausgebildeten Lichtaustrittsstellen mit ihren Längsmittelachsen schräg zur Längsmittelachse der im Mittelpunktbereich angeordneten Lichtaustrittsstelle ausgerichtet. Hierzu kann der Ringkörper bevorzugt einen schräg zur Längsmittelachse und Rückseite geneigten Ringkörperabschnitt aufweisen an oder in dem die auf der Kreislinie ausgebildeten Lichtaustrittsstellen zu einer geneigten Ausrichtung angeordnet sind.

Weiter ist vorgesehen, daß die im Mittelpunktsbereich der Kreislinie angeordnete Lichtaustrittsstelle durch einen um 45 Grad zur Mittellängsachse des Ringkörpers geneigten und durch ein Trägerrohr mit dem Ringkörper verbundenen Umlenkspiegel gebildet ist, vor dem das eine Ende eines optischen Lichtleiters endet, dessen anderes Ende der gemeinsamen Lichtquelle im Gehäuse zugeordnet ist.

Für die Führung der Lichtleiter ist vorgesehen, den Ringkörper durch ein rohrförmiges Halteglied mit dem die Lichtquelle aufnehmenden Gehäuse fest oder verstellbar zu verbinden und die optischen Lichtleiter in der Mittelöffnung des Haltegliedes anzuordnen. Es versteht sich, daß die Lichtleiter auch anderweitig, z.B. außerhalb des rohrförmigen Haltegliedes zwischen Gehäuse und Lichtaustrittsstellen verlegt sein können.

In weiterer Ausbildung der Vorrichtung ist vorgesehen, den Eintrittsenden der optischen Lichtleiter einen im Gehäuse untergebrachten und in die Strahlung der Lichtquelle einschwenk oder einschiebbaren optischen Filter und/oder eine durchscheinende bzw. durchsichtige Farbscheibe zuzuordnen. Filter und Farbscheibe sind geeignet, die Beurteilung der Interferenzmuster zu erleichtern.

Als Lichtquelle kann fernerhin eine Glühlampe, z.B. eine Halogenlampe dienen. Zweckmäßig soll die Helligkeit der Lichtquelle veränderbar sein, wozu bevorzugt die Glühlampe vermittels eines im Stromkreis derselben angeordneten veränderlichen Widerstand beeinflußbar ist. Auch besteht die Möglichkeit, die Glühlampe vermittels eines elektronischen Regelkreises in der Helligkeit zu verändern.

Schließlich ist in Abwandlung der Vorrichtung noch vorgesehen, den Lichtaustrittsstellen im Ringkörper angeordnete getrennte elektrische Lichtquellen zuzuordnen, die jeweils über elektrische Leiter mit einer Stromversorgungs- und Regeleinrichtung in Verbindung stehen. Auch besteht zum Erhalt einer Lichtstrahlung an den Lichtaustrittsstellen die Möglichkeit, daß das Gehäuse eine der Anzahl der Lichtaustrittsstellen im Ringkörper entsprechende Anzahl unabhängige Lichtquellen aufweist, die getrennt über optische Leiter an den Lichtaustrittsstellen optisch ausmünden.

Die Erfindung ist anhand eines Ausführungsbeispiels in der Zeichnung verdeutlicht. Es zeigen:
- Fig. 1: eine Vorrichtung in Vorderansicht,
- Fig. 2: einen Ringkörper in Seitenansicht,
- Fig. 3: einen Ringkörper perspektivisch, vergrößert,
- Fig. 4: eine Vorrichtung ausgerichtet auf ein Auge schematisch und
- Fig. 5: eine Vorrichtung bekannter Ausführung ausgerichtet auf ein Auge, schematisch.

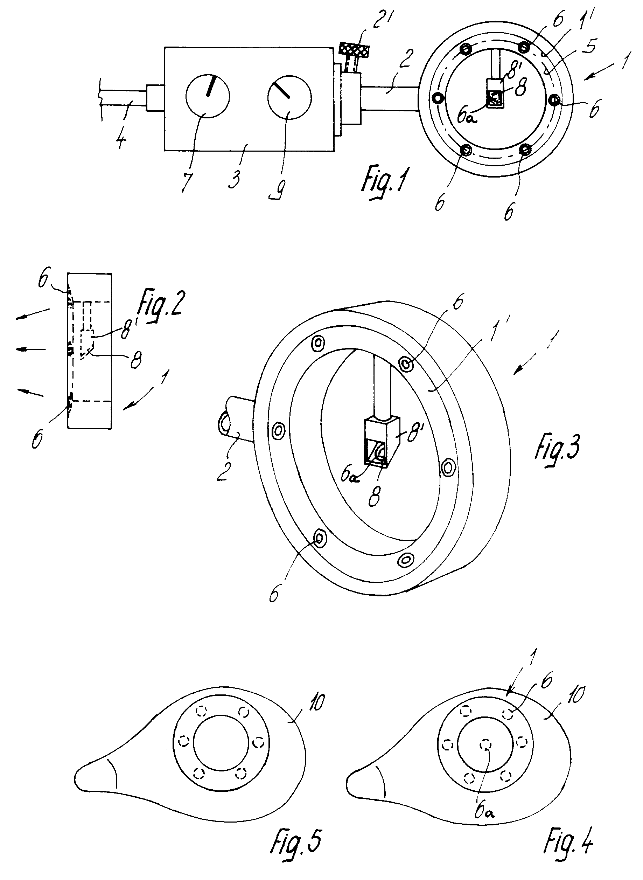

In den Fig. ist mit 1 ein Ringkörper bezeichnet, der durch ein rohrförmiges Halteglied 2 mit einem Gehäuse 3 durch eine Klemmschraube 2' verbunden ist, das zur Aufnahme einer Lichtquelle, z.B. Glühlampe (nicht gezeigt) dient. Mit 4 ist ein Zuführungskabel zur Glühlampe bezeichnet. Der Ringkörper 1 nimmt auf einer Kreislinie 5 sechs um gleiche Winkelgrade zueinander versetzte Lichtaustrittsstellen 6 sowie im Mittelpunktbereich der Kreislinie 5 eine weitere siebte Lichtaustrittsstelle 6a auf. Die Lichtaustrittsstellen 6 sind im wesentlichen punktförmig ausgebildet. Die Lichtaustrittsstellen 6 stehen über im Ringkörper 1 und dem Rohrstück 2 geführte optische Lichtleiter (nicht gezeigt) mit der im Gehäuse 3 untergebrachten Lichtquelle in Verbindung, sodaß bei Betätigung eines Ausschalters (nicht gezeigt) vermittels des Betätigungsgliedes 7 eine Lichtstrahlung an den Lichtaustrittsstellen 6 erfolgt. Die Strahlung der auf der Kreislinie 5 des Ringkörpers 6 angeordneten Lichtaustrittsstellen 6 erstreckt sich beim Ausführungsbeispiel schräg zur Mittellängsachse der im Mittelpunktbereich angeordneten Lichtaustrittsstelle 6a. Sie sind im Bereich eines Ringkörperabschnitts 1' angeordnet, der zur Mittellängsachse des Ringkörpers 6 und rückwärts geneigt ist. Die Lichtaustrittsstelle 6a im Mittelpunktbereich ist durch einen Umlenkspiegel 8 erreicht, der in einem Spiegelgehäuse 8' um 45 Grad zur Mittellängsachse ausgerichtet ist und die Strahlung eines optischen Lichtleiters nach vorne abgibt (Fig. 2). Das Betätigungsglied 7 kann eine Regeleinrichtung für die Helligkeit der Lichtquelle, z.B. einen Dreh- oder Schiebewiderstand, beeinflussen.

Mit 9 ist ein weiteres Betätigungsglied für einen in die Strahlung der Lichtquelle einschwenkbaren Licht- oder Farbfilter, insbesondere Blaufilter bezeichnet, der entsprechend seiner Stellungen aus der Lampenstrahlung heraus befördert bzw. in diese einbringbar ist.

In Fig. 4 ist die Zuordnung der Vorrichtung zu einem Auge 10 erkennbar. Vermittels der Siebenpunktstrahlung ist die Beurteilung des Tränenfilms auch an seiner dünnsten Stelle möglich. Entsprechend dem Erscheinungsbild der dabei erhaltenen Interferenzmuster ist der Tränenfilm in Gruppen aufteilbar und eine Abstimmung des für den jeweiligen Tränenfilm mit dem für ihn am besten geeigneten Kontaktlinsenmaterial möglich. Auf diese Weise ist die Anpaßzeit der Kontaktlinsen stark verkürzt. Außerdem ist ein längerer Anpaßerfolg damit verbunden.

Die bekannte Vorrichtung der Fig. 5 erzeugt ein Sechspunktringlicht auf einem zugeordneten Auge 10, das dabei lediglich eine Beurteilung des Tränenfilms an Stellen mit relativ großer Gleichmäßigkeit erlaubt.

## Patentansprüche

1. Beleuchtungsvorrichtung für die Untersuchung des Tränenfilms eines Auges, mit einem eine Mittelöffnung aufweisenden Trägerkörper (1), in dem auf einer Kreislinie (5) um die Mittelöffnung herum in gleichmäßigem Winkelabstand Lichtaustrittsstellen (6) so angeordnet sind, daß mit dem durch diese austretenden Licht das zu untersuchende Auge beleuchtet werden kann,
dadurch gekennzeichnet, daß innerhalb der Mittelöffnung des Trägerkörpers (1) im Bereich des Mittelpunkts der Kreislinie (5) eine Einrichtung mit einer weiteren, im wesentlichen punktförmigen Lichtaustrittsstelle (6a) zur Beleuchtung des Auges angeordnet ist.

2. Vorrichtung nach Anspruch 1, dadurch gekennzeichnet, daß der Trägerkörper (1) durch einen Ringkörper gebildet ist.

3. Vorrichtung nach Anspruch 1 und 2, dadurch gekennzeichnet, daß der Ringkörper (1) auf der Kreislinie (5) sechs und im Mittelpunktsbereich der Kreislinie (5) eine Lichtaustrittsstelle (6a) aufweist.

4. Vorrichtung nach Anspruch 2, dadurch gekennzeichnet, daß der Ringkörper (1) durch ein Halteglied (2) an einem eine Lichtquelle für die Lichtaustrittsstellen (6,6a) aufweisenden Gehäuse (3) festgelegt ist und daß die Strahlung der Lichtquelle über optische Lichtleiter den Lichtaustrittsstellen (6,6a) des Ringkörpers (1) zuführbar ist.

5. Vorrichtung nach Anspruch 1 und 2, dadurch gekennzeichnet, daß die auf der Kreislinie (5) ausgebildeten Lichtaustrittsstellen (6) mit ihren Längsmittelachsen schräg zur Längsmittelachse der im Mittelpunktsbereich angeordneten Lichtaustrittsstelle (6a) ausgerichtet sind.

6. Vorrichtung nach Anspruch 5, dadurch gekennzeichnet, daß der Ringkörper (1) auf einer Stirnseite einen schräg zur Längsmittelachse und Rückseite geneigten Ringkörperabschnitt (1') aufweist und daß die auf der Kreislinie (5) ausgebildeten Lichtaustrittsstellen am oder in dem geneigten Ringkörperabschnitt (1') angeordnet sind.

7. Vorrichtung nach Anspruch 1 und 4, dadurch gekennzeichnet, daß die im Mittelpunktsbereich der Kreislinie (5) angeordnete Lichtaustrittsstelle (6a) durch einen um 45° Grad zur Mittellängsachse des Ringkörpers (1) geneigten und durch ein Trägerrohr und Gehäuse (8) mit dem Ringkörper (1) verbundenen Umlenkspiegel (8) gebildet ist, vor dem das eine Ende eines optischen Lichtleiters endet, dessen anderes Ende der gemeinsamen Lichtquelle im Gehäuse (3) zugeordnet ist.

8. Vorrichtung nach Anspruch 4, dadurch gekennzeichnet, daß der Ringkörper (1) durch ein rohrförmiges Halteglied (2) mit dem die Lichquelle aufnehmenden Gehäuse (3) fest oder verstellbar fest verbunden ist und daß die optischen Lichtleiter durch die Mittelöffnung des Haltegliedes (2) hindurchgreifen.

9. Vorrichtung nach Anspruch 1, 4, 7 und 8, dadurch gekennzeichnet, daß den Eintrittsenden der optischen Lichtleiter ein im Gehäuse (3) angeordneter und in die Strahlung der Lichtquelle einbringbarer bzw. aus der Strahlung der Lichtquelle entfernbarer optischer Filter und/oder eine durchscheinende bzw. durchsichtige Farbscheibe zugeordnet ist.

10. Vorrichtung nach Anspruch 1 und 4, dadurch gekennzeichnet, daß die Lichtquelle durch eine Glühlampe gebildet ist.

11. Vorrichtung nach Anspruch 10, dadurch gekennzeichnet, daß die Helligkeit der Glühlampe veränderbar ist.

12. Vorrichtung nach Anspruch 11, dadurch gekennzeichnet, daß die Glühlampe vermittels eines im Stromkreis derselben angeordneten veränderlichen Widerstandes in der Helligkeit veränderbar ist.

13. Vorrichtung nach Anspruch 11, dadurch gekennzeichnet, daß die Glühlampe vermittels eines elektronischen Regelkreises in der Helligkeit veränderbar ist.

14. Vorrichtung nach Anspruch 1, dadurch gekennzeichnet, daß den Lichtaustrittsstellen (6) des Ringkörpers (1) im Ringkörper unabhängig ausgebildete elektrische Lichtquellen zugeordnet sind, die über elektrische Leiter mit einer Stromversorgungs- oder Stromversorgungs- und Regeleinrichtung, z.B. im Gehäuse (3) in Verbindung stehen.

15. Vorrichtung nach Anspruch 1 und 4, dadurch gekennzeichnet, daß das Gehäuse (3) eine der Anzahl der Lichtaustrittsstellen (6a) im Ringkörper (1) entsprechende Anzahl Lichtquellen aufweist, die getrennt über optische Lichtleiter mit den Lichtaustrittsstellen (6a) im Ringkörper (1) in Verbindung stehen.

## Claims

1. Illuminating device for examining the lacrimal film of an eye, having a support member (1), which is provided with a central aperture and in which light-emitting locations (6) are so disposed on a circle (5) around the central aperture, with uniform angular spacings therebetween, that the eye to be examined can be illuminated with the light emerging through said locations, characterised in that a means having an additional, substantially punctiform light-emitting location (6a) for illuminating the eye is disposed internally of the central aperture of the support member (1) in the region of the centre of the circle (5).

2. Device according to claim 1, characterised in that the support member (1) is formed by an annular member.

3. Device according to claims 1 and 2, characterised in that the annular member (1) has six light-emitting locations on the circle (5) and one light-emitting location (6a) in the central region of the circle (5).

4. Device according to claim 2, characterised in that the annular member (1) is secured, by a retaining member (2), on a housing (3) which includes a light source for the light-emitting locations (6, 6a), and in that the radiation of the light source can be supplied to the light-emitting locations (6, 6a) of the annular member (1) via optical photoconductors.

5. Device according to claims 1 and 2, characterised in that the light-emitting locations (6), which are provided on the circle (5), are orientated with their central longitudinal axes in an inclined manner relative to the central longitudinal axis of the light-emitting location (6a) disposed in the central region.

6. Device according to claim 5, characterised in that the annular member (1) is provided, on an end face, with a portion (1'), which slopes in an inclined manner relative to the central longitudinal axis and rear side, and in that the light-emitting locations, provided on the circle (5), are disposed on or in the inclined portion (1') of the annular member.

7. Device according to claims 1 and 4, characterised in that the light-emitting location (6a), which is disposed in the central region of the circle (5), is formed by a mirror (8), which is inclined by 45° relative to the longitudinal central axis of the annular member (1) and connected to the annular memer (1) by means of a carrier tube and housing (8'), one end of an optical photoconductor terminating in front of said mirror, and the other end of said photoconductor being associated with the common light source in the housing (3).

8. Device according to claim 4, characterised in that the annular member (1) is securedly or displaceably securedly connected to the housing (3), accommodating the light source, by means of a tubular retaining member (2), and in that the optical photoconductors extend through the central aperture in the retaining member (2).

9. Device according to claims 1 4, 7 and 8, characterised in that an optical filter, which is disposed in the housing (3) and can be inserted into the radiation of the light source, or respectively can be removed from the radiation of the light source, and/or a translucent or transparent colour screen and/is associated with the inlet ends of the optical photoconductors.

10. Device according to claims 1 and 4, characterised in that the light source is formed by an incandescent lamp.

11. Device according to claim 10, characterised in that the brightness of the incandescent lamp is variable.

12. Device according to claim 11, characterised in that the incandescent lamp is variable in respect of its brightness by means of a variable resistor disposed in the circuit of said lamp.

13. Device according to claim 11, characterised in that the incandescent lamp is variable in respect of its brightness by means of an electronic control circuit.

14. Device according to claim 1, characterised in that electric light sources are associated with the light-emitting locations (6) of the annular member (1), said light sources being independently provided in the annular member and being connected, via electrical conductors, to a power supply or power supply and control means, e.g. in the housing (3).

15. Device according to claims 1 and 4, characterised in that the housing (3) has a number of light sources corresponding to the number of light-emitting locations (6a) in the annular member (1), said light sources being connected separately, via optical photoconductors, to the light-emitting locations (6a) in the annular member (1).

## Revendications

1. Dispositif d'illumination pour analyser le film de larmes d'un oeil, comportant un corps porteur (1) présentant une ouverture centrale, dans lequel sont agencées sur une ligne circulaire (5) autour de l'ouverture centrale avec un écartement angulaire uniforme des zones de sortie de lumière (6) de sorte que, par la lumière sortant de ces zones, l'oeil à analyser peut être illuminé,
caractérisé en ce qu'à l'intérieur de l'ouverture centrale du corps porteur (1), dans la zone du centre de la ligne circulaire (5), est agencé un dispositif comportant une autre zone de sortie de lumière (6a) pour l'essentiel de forme ponctuelle, pour l'illumination de l'oeil.

2. Dispositif selon la revendication 1,
caractérisé en ce que le corps porteur (1) est formé d'un corps annulaire.

3. Dispositif selon les revendications 1 et 2,
caractérisé en ce que le corps annulaire (1) comporte sur la ligne circulaire (5) six zones de sortie de lumière et dans la zone du centre de la ligne circulaire (5) une zone de sortie de lumière (6a).

4. Dispositif selon la revendication 2,
caractérisé en ce que le corps annulaire (1) est fixé par un élément de maintien (2) sur un boîtier (3) comportant une source de lumière pour les zones de sortie de lumière (6,6a), et en ce que le rayonnement de la source de lumière peut être amené par l'intermédiaire de guides optiques de lumière aux zones de sortie de lumière (6,6a) du corps annulaire (1).

5. Dispositif selon les revendications 1 et 2,
caractérisé en ce que les zones de sortie de lumière (6) formées sur la ligne circulaire (5) sont dirigées par leur axe central longitudinal de façon inclinée par rapport à l'axe central longitudinal de la zone de sortie de lumière (6a) agencée dans la zone du centre.

6. Dispositif selon la revendication 5,
caractérisé en ce que le corps annulaire (1) présente sur une face frontale un tronçon (1') de corps annulaire incliné par rapport à l'axe central longitudinal et à la face arrière, et en ce que les zones de sortie de lumière formées sur la ligne circulaire (5) sont agencées sur ou dans le tronçon (1') de corps annulaire incliné.

7. Dispositif selon les revendications 1 et 4,
caractérisé en ce que la zone de sortie de lumière (6a) agencée dans la zone du centre de la ligne circulaire (5) est formée d'un miroir de renvoi (8) incliné de 45° par rapport à l'axe longitudinal central du corps annulaire (1) et relié par un tube porteur et un boîtier (8') au corps annulaire (1), devant lequel miroir de renvoi s'arrête l'une des extrémités d'un guide optique de lumière, dont l'autre extrémité est associée à la source de lumière commune dans le boîtier (3).

8. Dispositif selon la revendication 4,
caractérisé en ce que le corps annulaire (1) est relié, de façon solidaire ou de façon à pouvoir être réglé de manière solidaire, par un élément de maintien (2) en forme de tube au boîtier (3) recevant la source de lumière, et en ce que le guide optique de lumière traverse l'ouverture centrale de l'élément de maintien (2).

9. Dispositif selon les revendications 1, 4, 7 et 8,
caractérisé en ce qu'aux extrémités d'entrée des guides optiques de lumière sont associés un filtre optique agencé dans le boîtier (3) et pouvant être amené dans le rayonnement de la source de lumière ou être écarté du rayonnement de la source de lumière et/ou un écran coloré translucide ou transparent.

10. Dispositif selon les revendications 1 et 4,
caractérisé en ce que la source de lumière est formée d'une lampe à incandescence.

11. Dispositif selon la revendication 10,
caractérisé en ce que la luminosité de la lampe à incandescence peut être réglée.

12. Dispositif selon la revendication 11,
caractérisé en ce que la lampe à incandescence est réglable en luminosité au moyen d'une résistance variable agencée dans le circuit électrique de la lampe à incandescence.

13. Dispositif selon la revendication 11,
caractérisé en ce que la lampe à incandescence est réglable en luminosité au moyen d'un circuit de réglage électronique.

14. Dispositif selon la revendication 1,
caractérisé en ce qu'aux zones de sortie de lumière (6) du corps annulaire (1) sont associées des sources de lumière électriques formées de façon indépendante dans le corps annulaire et reliées par l'intermédiaire de conducteurs électriques à un dispositif d'alimentation en courant ou un dispositif d'alimentation en courant et de réglage, par exemple dans le boîtier (3).

15. Dispositif selon les revendications 1 et 4,
caractérisé en ce que le boîtier (3) comporte un nombre de sources de lumière correspondant au nombre de zones de sortie de lumière (6a) dans le corps annulaire (1), sources de lumière qui sont reliées de façon séparée par l'intermédiaire de guides optiques de lumière aux zones de sortie de lumière (6a) dans le corps annulaire (1).
